# EUROPEAN PATENT SPECIFICATION

(11) **EP 0 563 301 B1**
(45) Date of publication and mention of the grant of the patent: **10.05.2000**
(21) Application number: 92903695.2
(22) Date of filing: 19.12.1991
(51) Int. Cl.: A61K 7/00, A61K 31/42

(54) **Cosmetic Process of Reducing the Rate of Mammalian Hair Growth**
Kosmetisches Verfahren zur Verringerung der Haarwuchsrate bei Säugetieren
Procédé cosmétique pour la réduction du taux de croissance des poils chez les mammifères

(30) Priority: 20.12.1990 US 632126
(43) Date of publication of application: 06.10.1993
(73) Proprietor: HANDELMAN, Joseph H., New York, NY 10023 (US)
(72) Inventor: HANDELMAN, Joseph, H., New York, NY 10023 (US); SHANDER, Douglas, Gaithersburg, MD 20878 (US); FUNKHOUSER, Margaret, G., Arlington, VA 2204 (US)
(74) Representative: Baillie, Iain Cameron
(86) International application number: US9109645
(87) International publication number: WO9211007

(56) References cited:
- US-A- 4 720 489
- US-A- 4 912 120
- HATTORI, M. et al., "Biochemical Analysis of Hair Growth From the Aspects of Aging and Enzyme Activities", J. Derm. 10(1): 45-54. (1983).
- MARTINET, N. et al., "Epidermal and Hair Follicle Transglutaminases", J. Biol. chem. 263(9): 4236-41. (1988).
- OGAWA H. et al., "Regulation Mechanisms of Hair Growth", Curr. Prob. Derm." (Norm. Abnorm. Epiderm. Diff.): 159-70 (1983) Abstract.
- GOLDSMITH, L. A., "Hair Follicle Transglutaminases and the Formation of -( -glutamyl) Cross-links", Hair Research. Springer-Verlag 1981, pp 29-34.
- CHUNG, S.I. et al., "Transglutaminase from Hair Follicle of Guinea Pig", Proc. Natl. Sci., USA 69(2): 303-307 (1972).
- KILLACKY, J.J.F. et al., "A New Class of Mechanism-Based Inhibitors of Transglutaminase Enzymes Inhibits the Formation of Cross-Linked Envelopes By Human Malignant Keratinocytes", Mol. Pharmacol. 35: 701-706 (1989).
- HARDING, H.W.J. et al., "Formation of the -( -Glutamyl) lysine Cross-Link in Hair Proteins. Investigation of Transamindases in Hair Follicles", Biochem 11(15): 2858-63 (1972).
- PETERSON, L.L. et al., "Rat Hair Eollicle and Epidermal Transglutaminases Biochemical and Immunochemical Isoenzymes" Biochim. Biophys Acta 657: 268-76 (1981).
- OGAWA H. et al., "Human Epidermal Transglutaminase", J. Invest. Derm. 68: 32-35 (1977).

## Description

This invention relates to a cosmetic method for altering the rate of mammalian hair growth, particularly androgen-stimulated hair growth, by topical application to the skin of a composition containing an inhibitor of transglutaminase.

The enzyme transglutaminase (TGase (R-glutamylpeptide: amine gamma-glutamyl-transferase EC 2.3.2.13) has been reported to catalyze the formation of dipeptide bonds between the glutamine and lysine residues in polypeptides via covalent coupling of the gamma carboxyamide group of peptide bound glutamine residues with an epsilon amino group of peptide bound lysine residues (Folk, et al., Adv. Enzymol., 38:109-191 (1973)). The TGase in hair follicles of humans is reported to be distinct from the epidermal TGase (Ogawa, et al., J. Invest. Dermatol., 68:32-35 (1977)). TGase is also said to be found in hair follicles of guinea pigs (Chung, et al., Proc. Natl. Acad. Sci., USA 69:303-307 (1972)); of rats (Peterson, et al., Biochim. Biophys. Acta, 657:268-276 (1981)); and of sheep (Harding, et al., Biochemistry, 11:2858-2863 (1972)).

In addition to direct detection of TGases in follicles, the dipeptide gamma-glutaminyl (lysyl) crosslinks have also been identified in the proteins of the medulla of the hair shaft and the inner root sheath of guinea pig follicle which adjoins the cortical cells of the hair (Harding, et al., Biochemistry, 10,(4):624-630 (1971). These dipeptide bonds are said to stabilize structural proteins imparting structural integrity to the medulla and to provide resistance to proteolytic attack by microorganisms (Goldsmith, L.A., "Hair Follicle Transglutaminases and the Formation of -(Glutamyl) Cross Links in Hair Research", pp. 290-35, 1981, Ed. by Orfanos Montana, and Stuttgen, Springer Verlag Berlin Heidelberg).

The enzyme activity of TGase is reported to be increased in actively growing versus quiescent regions of hair growth Hattori, et al., J. of Dermatology, 10:45-54 (1983)). It has not been known what impact, if any inhibition of transglutaminase activity would have on the rate or character of hair growth since its activity on hair shaft proteins is confined to the latter stages of hair growth involving the secondary or posttranslational modifications of previously synthesized shaft proteins.

U.S. Patents Castelhano et al. 4,912,120 and 4,929,630 describe various 3,5-disubstituted-4,5-dihydroisoxazoles as inhibitors of transglutaminase useful for administration to mammals suffering from a disease characterized by elevated transglutaminase activity, such as acne, psoriasis, or cataracts.

It has previously been proposed to alter the rate and character of hair growth by applying to the skin inhibitors of certain enzymes such as inhibitors of 5-alpha-reductase or of ornithine decarboxylase, or such antiandrogen materials as cytoplasmic androgen receptor binding agents, as described in U.S. Patents Nos. 4,720,489 and 4,885,298. Moreover, it has been theorized that other enzymes, including gamma-glutamyl transpeptidase, are involved in various stages of hair follicle formation or of hair growth, but the relation between the various enzymes and the reactions which they control, as well as their effect upon each other and upon hair growth, has not been fully understood, as appears from Richards et al., Cancer Research, 42:4143-4152 (1982); DeYoung et al, Cancer Research, 38:3697-3701 (1978); and Chase, Physiolo. Zool., 24:1-8 )1951)

In accordance with the present invention there is provided a cosmetic process of reducing the rate of mammalian hair growth which comprises the step of applying to the skin of mammals free from a disease characterized by elevated transglutaminase activity a composition containing an inhibitor of transglutaminase at a dosage effective to reduce the rate of said hair growth.

It has now been found that topical application to the skin of a normal mammal (including human), i.e., a mammal free from a disease characterized by elevated transglutaminase activity, of a composition containing an inhibitor of transglutaminase, is effective to reduce the rate and alter the character of mammalian hair growth, particularly androgen-stimulated hair growth. By "alter the character" is meant that it renders the hair, particularly androgen-stimulated hair growth, softer and downier and/or more easily cut. Particularly preferred is a process in which the inhibitor is a specific, non-competitive inhibitor of transglutaminase such as 3-bromo-5-(N-benzyloxycarbonyl-1-phenylalanamido-methyl)-4,5-dihydroisoxazole (BBD) or other 3,5-disubstituted-4,5-dihydroisoxazole inhibitor as described in U.S. Patents 4,912,120 and 4,929,630

The composition of the present invention contains, in addition to the inhibitor, a non-toxic dermatologically acceptable vehicle or carrier which is adapted to be spread upon the skin including the follicles. The concentration of the inhibitor in the composition may be varied over a wide range up to a saturated solution, preferably from 0.1 to 20% by weight; the reduction of hair growth increases as the amount of inhibitor applied increases per unit area of skin. The maximum amount effectively applied is limited only by the rate at which the inhibitor penetrates the skin. Generally, the effective amounts range from 10 to 2500 micrograms per square centimeter of skin. In the case of BBD inhibitor, which is hydrophobic, non-aqueous vehicles, such as those based on an alcohol, e.g., ethyl alcohol or benzyl alcohol, are preferred.

### Example 1

A vehicle or carrier was prepared having the following composition:

| | Percent by Volume |
|---|---|
| Acetone | 75% |
| Propylene Carbonate | 20% |
| Benzyl Alcohol | 5% |

The compound BBD was mixed with separate portions of the foregoing vehicle to provide specimens containing 1,2,5,10, and 20% by weight of the inhibitor respectively.

Five groups (seven or eight animals in each group) of male intact Golden Syrian hamsters were provided. These animals are considered acceptable models for human beard hair growth in that they display oval shaped flank organs, one on each side, each about 8 mm. in major diameter, which grow thick black and coarse hair similar to human beard hair. These organs produce hair in response to androgens in the hamster. The flank organs of each hamster were depilated by applying a thioglycolate-based chemical depilatory (Surgex), and to one organ of each animal was applied 10-25 µl. of vehicle alone once a day, while to the other organ of each animal was applied an equal amount of vehicle containing inhibitor. After 2 1/2 weeks of such applications (five days a week for a total of eighteen days), the flank organs were shaved and the amount of recovered hair (hair mass) from each was weighed. The extent of reduction in hair growth by the inhibitor was expressed as the percent decrease in hair mass on the organ treated with inhibitor as compared to the organ treated with vehicle alone. As a control, one additional control group of eight animals had both flank organs of each animal treated with vehicle alone. The results were as shown in Table 1 below.

**Table 1**

| **Inhibition of Hair Mass by the Transglutaminase Inhibitor 5- (N-Benzyloxycarbonyl-L-phenylalaninamidomethyl)-3-bromo-4,5-dihydroisoxazole (BBD)** Flank Organ Hair Mass (mg ± SEM) | | | | |
|---|---|---|---|---|
| Treatment | No. of Animals | Treated | Vehicle | %Inhibition (+SEM) |
| 1% BBD | 8 | 2.614 ± .19 | 3.289 ± .22 | 19.54 ± 6.07 |
| 2% BBD | 8 | 2.827 ± .23 | 2.881 ± .32 | -3.63 ± 9.91 |
| 5% BBD | 8 | 2.279 ± .28 | 3.021 ± .18 | 22.83 ± 9.62 |
| 10% BBD | 8 | 1.389 ± .12 | 3.207 ± .14 | 56.08 ± 4.64 |
| 20% BBD | 7 | .333 ± .06 | 2.743 ± .29 | 87.87 ± 1.54 |
| Control | 8 | 3.146 ± .24 | 3.239 ± .36 | -1.76 ± 6.98 |

Visual inspection of the hamsters during treatment showed that the inhibitor caused a decrease in the length of the hair dependent upon the concentration of the inhibitor. Microscopic examination of representative hair shafts harvested from the flank organs showed that treatment with the 20% concentration of BBD produced a dramatic deterioration of the hair shaft structure including disorganization of the medulla and cuticle; less dramatic but visually obvious changes in the medulla of hair shafts from organs treated with 5% and 10% concentrations were also observed. It was also found that the inhibiting effect of BBD on transglutaminase activity was dose related.

## Claims

1. A cosmetic process of reducing the rate of mammalian hair growth which comprises the step of applying to the skin of mammals free from a disease characterized by elevated transglutaminase activity a composition containing an inhibitor of transglutaminase at a dosage effective to reduce the rate of said hair growth.

2. The process as claimed in claim 1, in which said inhibitor is 5-(N-benzyl-oxycarbonyl-1-phenylalanamidomethyl)-3-bromo-4,5-dihydroisoxazole.

3. The process as claimed in claim 1 or 2, in which said dosage is from 10 to 2500 micrograms of said inhibitor per square centimeter of skin.

4. The process as claimed in claim 1, in which said inhibitor is a 3,5-disubstituted-4,5-dihydroisoxazole.

## Patentansprüche

1. Kosmetisches Verfahren zur Verminderung der Geschwindigkeit des Haarwuchses von Mammalia, welches Verfahren den Schritt des Aufbringens einer Zusammensetzung auf die Haut von Mammalia umfasst, die frei sind von Erkrankungen, die durch erhöhte Transglutaminase-Aktivität gekennzeichnet sind, welche Zusammensetzung einen Inhibitor für Transglutaminase in einer Dosierung enthält, die wirksam ist, um die Geschwindigkeit des Haarwuchses zu vermindern.

2. Verfahren nach Anspruch 1, wobei der Inhibitor 5-(N-Benzyloxycabonyl-1-phenylalaninamidomethyl)-3-brom-4,5-dihydroisoxazol ist.

3. Verfahren nach Anspruch 1 oder 2, wobei die Dosierung 10 ... 25 Mikrogramm des Inhibitors pro Quadratzentimeter Haut beträgt

4. Verfahren nach Anspruch 1, wobei der Inhibitor ein 3,5-substituiert-4,5-Dihydroisoxazol ist.

## Revendications

1. Procédé cosmétique de réduction de la vitesse de la pousse des poils des mammifères comprenant l'étape d'application sur la peau de mammifères ne présentant pas de pathologie se caractérisant par une activité élevée de transglutaminase, une composition contenant un inhibiteur de transglutaminase à une dose efficace pour réduire la vitesse de ladite pousse des poils.

2. Procédé selon la revendication 1, dans lequel ledit inhibiteur est le 5-(N-benzyloxycarbonyl-1-phénylalanamidométhyl)-3-bromo-4,5-dihydroisoxazole.

3. Procédé selon la revendication 1 ou 2, dans lequel ladite dose est de 10 à 2500 microgrammes dudit inhibiteur par centimètre carré de peau.

4. Procédé selon la revendication 1, dans lequel ledit inhibiteur est un 4,5-dihydroisoxazole disubstitué en 3,5.
